# EUROPÄISCHE PATENTANMELDUNG

(11) **EP 4 286 357 A1**
(43) Veröffentlichungstag der Anmeldung: **06.12.2023**
(21) Anmeldenummer: 22020259.2
(22) Anmeldetag: 03.06.2022
(51) Int. Cl.: C07C 29/152, C07C 31/04, C01B 3/00

(54) **VERFAHREN UND ANLAGE ZUR HERSTELLUNG VON METHANOL**

(71) Anmelder: Linde GmbH, 82049 Pullach (DE); Technische Universität München, 80333 München (DE)
(72) Erfinder: Schwarzhuber, Josef, 82049 Pullach (DE); Winkler, Florian, 82049 Pullach (DE); Peschel, Andreas, 82049 Pullach (DE); Hemauer, Johanna, 80290 München (DE); Klein, Harald, 80290 München (DE)
(74) Vertreter: Fischer, Werner

(57) **Zusammenfassung**

Es wird ein Verfahren (100, 200, 300) zur katalytischen Herstellung von Methanol vorgeschlagen wobei ein Reaktorsystem (10) mit einer ersten Reaktionszone (11) und einer zweiten Reaktionszone (12) verwendet wird, wobei die erste Reaktionszone (11) und die zweite Reaktionszone (12) adiabat betrieben werden und jeweils eine katalytische Reaktionszone (11) aufweisen, wobei zumindest ein Teil eines aus der ersten Reaktionszone (11) abströmenden Gasgemischs in die zweite Reaktionszone (12) überführt wird, wobei ein erster Einsatzstrom auf einem ersten Temperaturniveau stromauf der ersten Reaktionszone (11) in das Reaktorsystem (10) eingespeist wird, wobei ein zweiter Einsatzstrom auf einem zweiten Temperaturniveau zwischen der ersten Reaktionszone (11) und der zweiten Reaktionszone (12) in das Reaktorsystem (10) eingespeist wird, und wobei der erste Temperaturbereich oberhalb des zweiten Temperaturbereichs liegt. Eine entsprechende Anlage ist ebenfalls Gegenstand der vorliegenden Erfindung.

## Beschreibung

Die Erfindung betrifft ein Verfahren und eine Anlage zur Herstellung von Methanol.

### Hintergrund

Die Herstellung von Methanol erfolgt herkömmlicherweise ausgehend von Synthesegas. Hierbei werden zwei Moleküle Wasserstoff mit einem Molekül Kohlenmonoxid zu Methanol und, falls das Synthesegas Kohlendioxid enthält, drei Moleküle Wasserstoff mit einem Molekül Kohlendioxid zu einem Molekül Methanol und einem Molekül Wasser umgesetzt. Derartige Verfahren weisen eine hohe Reaktionsenthalpie auf, weshalb ein gekühlter Reaktor verwendet werden muss. Derartige Verfahren werden ferner typischerweise, aber nicht ausschließlich, in sehr großen Anlagen eingesetzt.

Die sogenannte "grüne" Methanolsynthese durch direkte Hydrierung von Kohlendioxid ist ebenfalls bekannt. Die Reaktionsgleichung ist dabei dieselbe wie im Fall der Reaktion von Kohlendioxid in Synthesegas. Limitationen ergeben sich aufgrund der Tatsache, dass es sich um eine Gleichgewichtsreaktion handelt und durch den Wasserpartialdruck. Allerdings entsteht bei entsprechenden Verfahren vorteilhafterweise weniger Abwärme aufgrund der geringeren Reaktionsenthalpie und es werden gegenüber der konventionellen Methanolsynthese geringere Mengen an Nebenprodukten gebildet.

Entsprechende Verfahren sind beispielsweise der sogenannte Camere-Prozess (vgl. Joo et al., CAMERE Process for methanol synthesis from CO2 hydrogenation, Studies in Surface Science and Catalysis 153:67-72, 2004), der eine reverse Wassergasshiftreaktion umfasst, bei der die Methanolsynthese jedoch unter Verwendung von Synthesegas durchgeführt wird. In diesen und anderen Verfahren können Rohrreaktoren eingesetzt werden, wie in der US 9,040,594 B2 offenbart.

Die vorliegende Erfindung stellt sich die Aufgabe, entsprechende Verfahren insbesondere im Hinblick auf Erstellungskosten und -aufwand zu verbessern.

### Offenbarung der Erfindung

Diese Aufgabe wird durch ein Verfahren und eine Anlage mit den jeweiligen Merkmalen der unabhängigen Patentansprüche gelöst. Vorteilhafte Ausgestaltungen der vorliegenden Erfindung sind Gegenstand der abhängigen Patentansprüche und der nachfolgenden Beschreibung.

Ist nachfolgend von einem Wasserstoff enthaltenden Frischeinsatz die Rede, soll hierunter nicht notwendigerweise ein reiner Wasserstoffstrom verstanden werden, sondern ggf. auch ein Wasserstoff enthaltendes Gasgemisch, das überwiegend Wasserstoff enthält, beispielsweise in einem Gehalt von mehr als 75%, 80%, 85%, 90% oder 95% im Volumen-, Masse- oder Molanteil. Entsprechend soll unter einem Kohlendioxid enthaltenden Frischeinsatz nicht notwendigerweise ein reiner Kohlendioxidstrom verstanden werden, sondern ggf. auch ein Kohlendioxidenthaltendes Gasgemisch, das überwiegend Kohlendioxid enthält, beispielsweise in einem Gehalt von mehr als 75%, 80%, 85%, 90% oder 95% im Volumen-, Masse- oder Molanteil. Ein Frischeinsatz unterscheidet sich dabei dadurch von einem Rückführstrom, dass er keine Anteile enthält, die zuvor bereits ein Reaktorsystem durchlaufen haben, dem der Frischeinsatz zugeführt wird.

Eine "Bildung" eines Stoffstroms kann in beliebiger Weise erfolgen, beispielsweise durch Abzweigen von einem Haupt- oder Ausgangsstrom oder Vereinigen eines ersten und eines zweiten Stoffstroms. Eine "Bildung" eines Stoffstroms kann eine Druckbeaufschlagung, eine Abkühlung, eine Erwärmung, eine Verdampfung, ein Austreiben von Komponenten oder eine Zugabe von Komponenten umfassen. Ist hier davon die Rede, dass ein zweiter Stoffstrom "unter Verwendung" eines ersten Stoffstroms "gebildet" wird, kann dies auch bedeuten, dass der erste Stoffstrom als der zweite Stoffstrom verwendet wird und beispielsweise einer anderen Funktion zugeführt wird, der erste und der zweite Stoffstrom also im Übrigen identisch sind.

Im Rahmen der Erfindung wird ein Verfahren zur katalytischen Herstellung von Methanol, insbesondere gemäß den eingangs erwähnten Reaktionsprinzipien, vorgeschlagen, wobei ein Reaktorsystem mit einer ersten Reaktionszone und einer zweiten Reaktionszone verwendet wird, wobei die erste Reaktionszone und die zweite Reaktionszone adiabat betrieben werden und jeweils als katalytische Reaktionszonen ausgebildet sind, wobei zumindest ein Teil eines aus der ersten Reaktionszone abströmenden Gasgemischs in die zweite Reaktionszone überführt wird, wobei ein erster Einsatzstrom auf einem ersten Temperaturniveau stromauf der ersten Reaktionszone in das Reaktorsystem eingespeist wird, wobei ein zweiter Einsatzstrom auf einem zweiten Temperaturniveau zwischen der ersten Reaktionszone und der zweiten Reaktionszone in das Reaktorsystem eingespeist wird, und wobei der erste Temperaturbereich oberhalb des zweiten Temperaturbereichs liegt.

Die vorliegende Erfindung geht von der Situation aus, dass die für die "grüne" Methanolproduktion, wie sie eingangs erläutert wurde, verwendeten Reaktoren derzeit vergleichsweise klein sind, d.h. diese sind typischerweise für eine Tagesproduktion von deutlich unter 1.000 Tonnen Methanol eingerichtet. Dies gilt insbesondere im Vergleich zu konventionellen Anlagen, in denen die Tagesproduktion typischerweise bei bis zu ca. 6.000 Tonnen pro Tag liegt. Insbesondere bei kleineren Anlagen schlagen dabei die Erstellungskosten (CAPEX) auf die Gesamtkosten stärker durch und sollten daher vorteilhafterweise reduziert werden.

Ein für diese Zielsetzung vorteilhafter Reaktor wird insbesondere adiabat betrieben. Typischerweise wird hier aber die Umsetzung zu Methanol aufgrund der hohen Reaktoraustrittstemperaturen und das hierdurch stärker zu den Edukten verschobene Reaktionsgleichgewicht beschränkt. Ferner begünstigen höhere Reaktoraustrittstemperaturen die Bildung von Nebenprodukten. Höhere Temperaturen verursachen ferner hohe Kohlenmonoxidgehalte in Rückführströmen aufgrund einer reversen Wassergasshiftreaktion. Mehr Kohlenmonoxid begünstigt zusätzlich die Bildung von Nebenprodukten.

Vorteile der vorliegenden Erfindung umfassen dabei insbesondere eine geringe Nebenkomponentenrate, da einerseits Kohlendioxid als Einsatz verwendet wird und insbesondere ein adiabater Betrieb, aber mit "Kalteinspeisung" des zweiten Stoffstroms in der erläuterten Weise, durchgeführt wird. Hierdurch ist eine hohe Katalysatorlaufzeit möglich. Durch den adiabaten Betrieb und den damit verbundenen Verzicht auf ein externes Kühlsystem ergeben sich geringere Investitionskosten und ein geringerer apparatetechnischer Aufwand, beispielsweise weil keine Rohrbündelreaktoren mit entsprechender Kühlung bereitgestellt werden müssen. Der Prozess bzw. ein entsprechender Reaktor kann relativ flexibel betrieben werden (hinsichtlich dem Verhältnis von Wasserstoff und Kohlendioxid und der Gasraumgeschwindigkeit), was eine Anpassung an fluktuierende Verfügbarkeit von Wasserstoff möglich macht. Eine höhere Flexibilität ergibt sich insbesondere auch dadurch, dass zwei Feedströme in den Reaktor eingespeist werden.

Eine Zwischenkühlung und das entsprechende Auskondensieren von Wasser und Methanol in einem prinzipiell alternativ einsetzbaren Reaktorkonzept könnte zwar zur Erhöhung der Methanolausbeute verwendet werden, aber auch wiederum sehr hohe Erstellungs- und Betriebskosten (OPEX) verursachen. Entsprechendes gilt auch für die Verwendung von Rohrbündelreaktoren mit entsprechendem Kühlsystem.

Durch die erfindungsgemäß vorgeschlagene Zwischeneinspeisung des zweiten Einsatzstroms werden diese Nachteile überwunden, da die Zwischeneinspeisung eine Abkühlung des Prozessgases bzw. eine Verdünnung des heißen, aus der ersten Reaktionszone austretenden Prozessgases mit kühlerem Prozessgas bewirkt. Die erste und die zweite Reaktionszone können dabei insbesondere in einem gemeinsamen Druckbehälter angeordnet sein, wobei die Stromführung in den beiden Reaktionszonen insbesondere einander entgegengesetzt ist. Insbesondere kann ein Druckbehälter, insbesondere mit einem im wesentlichen zylinderförmigen Abschnitt vorgesehen sein, wobei die Reaktionszonen derart angeordnet sind, dass die erste Reaktionszone in Summe in einer ersten Richtung im Wesentlichen parallel zu der Zylinderachse des zylindrischen Abschnitts durchströmt wird und die zweite Reaktionszone in Summe in einer zweiten, der ersten Richtung entgegengesetzten Richtung parallel zu der Zylinderachse des zylindrischen Abschnitts durchströmt wird. Unter "in Summe in einer Richtung" soll dabei verstanden werden, dass das Gasvolumen in der ersten und der zweiten Reaktionszone sich insgesamt in eine entsprechende Richtung bewegt, einzelne Gasmoleküle aber auch abweichende Bewegungsrichtungen (bspw. durch Verwirbelungen und dergleichen oder eine Umlenkung an Partikeln eines Katalysatorbetts) aufweisen können. Die Einspeisung des ersten Einsatzstroms erfolgt dabei insbesondere in einem ersten terminalen Bereich des zylindrischen Abschnitts, die Einspeisung des zweiten Einsatzstroms insbesondere in einem dem ersten terminalen Bereich entgegengesetzten zweiten terminalen Bereich des zylindrischen Abschnitts.

Die beiden Reaktionszonen werden im Rahmen der vorliegenden Erfindung adiabat betrieben, worunter hier verstanden werden soll, dass den Reaktionszonen kein externes Kühlsystem zugeordnet ist. Die Einspeisung des zweiten Einsatzstroms bewirkt zwar auch eine Kühlung, stellt aber keine externe Kühlung dar, da die Komponenten des zweiten Einsatzstroms zu dem durch die Reaktionszonen geführten Prozessgasstrom zugespeist werden. Insbesondere wenn der zweite Reaktionseinsatzstrom kaltes oder verflüssigtes Gas (wie flüssiges Kohlendioxid) ist oder umfasst, kann eine besonders effektive Kühlung bewirkt werden, die ohne aufwendige und kostenintensive Maßnahmen erreicht werden kann. Auf diese Weise kann im Rahmen der vorliegenden Erfindung eine Erhöhung der Methanolausbeute erzielt werden, die insbesondere in den erwähnten kleineren Anlagen zur "grünen" Methanolproduktion ohne signifikant erhöhte Kosten erzielt werden kann.

Der erste Einsatzstrom enthält im Rahmen der vorliegenden Erfindung insbesondere überwiegend oder ausschließlich Wasserstoff und Kohlendioxid, wobei der Begriff "überwiegend" insbesondere für einen Gehalt von mehr als 75, 80, 85, 90 oder 95% auf Volumen- oder Stoffmengenbasis stehen kann.

In einer Ausgestaltung der vorliegenden Erfindung wird zumindest ein Teil eines aus der zweiten Reaktionszone abströmenden Gasgemischs unter Erhalt einer Gasfraktion und einer Flüssigfraktion einer Phasentrennung unterworfen, wobei unter Verwendung zumindest eines Teils der Gasfraktion ein Rückführstrom gebildet wird, und wobei der erste Einsatzstrom unter Verwendung des Rückführstroms gebildet wird.

Die Phasentrennung kann dabei in unterschiedlichen Ausgestaltungen der Erfindung unterschiedlich ausgeführt sein. Sie beruht stets auf einer Abkühlung des aus der zweiten Reaktionszone abströmenden Gasgemischs und einem Auskondensieren der dabei flüssig werdenden Komponenten, kann aber auch insbesondere ein Strippen der gebildeten Flüssigphase umfassen, wie nachfolgend erläutert. Die auskondensierenden Komponenten umfassen insbesondere Wasser, Methanol und zwischen Wasser und Methanol siedende Verbindungen, bspw. Ethanol, das als Nebenprodukt gebildet wird. Die Erfindung kann insbesondere die Verwendung nur eines Teils der Gasphase als Rückführstrom umfassen, wobei ein nicht zur Bildung des Rückführstroms verwendeter Teil aus dem Verfahren ausgeführt werden kann, um eine Akkumulation nicht umgesetzter Komponenten und/oder inerter Komponenten (z.B. Stickstoff) zu vermeiden (sog. Purge).

In einer Ausgestaltung der vorliegenden Erfindung wird dabei zumindest ein Teil des aus der zweiten Reaktionszone abströmenden Gasgemischs gegen zumindest einen Teil des ersten Einsatzstroms abgekühlt. Auf diese Weise schafft die vorliegende Erfindung eine vorteilhafte Wärmeintegration, mittels welcher der erste Einsatzstrom auf die zum Anspringen der Reaktion erforderliche Temperatur gebracht werden kann. Dies ist insbesondere vorteilhaft, wenn der erste Einsatzstrom sehr kalte Komponenten, wie beispielsweise einen flüssigen Kohlendioxidstrom, umfasst.

In einer Ausgestaltung der vorliegenden Erfindung wird der erste Einsatzstrom unter Verwendung eines Wasserstoff enthaltenden Frischeinsatzstroms gebildet. In dieser Ausgestaltung kann der erste Einsatzstrom insbesondere unter Verwendung des Wasserstoff enthaltenden Frischeinsatzstroms und des Rückführstroms gebildet werden. Alternativ kann der Wasserstoff enthaltende Frischeinsatzstrom in die Phasentrennung eingespeist werden, wobei zumindest ein Teil des Wasserstoffs in dem Wasserstoff enthaltenden Frischeinsatzstrom in der Phasentrennung in die Gasfraktion und hieraus in den Rückführstrom überführt wird.

Die erste Ausgestaltung umfasst also eine Einspeisung stromauf der ersten Reaktionszone oder, mit anderen Worten, eine Bildung des ersten Einsatzstroms unter Verwendung des Wasserstoff enthaltenden Frischeinsatzstroms, eines Rückführstroms und eines Kohlendioxid enthaltenden Frischeinsatzstroms wie unten erläutert. In der zweiten Ausgestaltung erfolgt die Einspeisung des Wasserstoff enthaltenden Frischeinsatzstroms dagegen stromab der zweiten Reaktionszone. In dieser zweiten Ausgestaltung kann dabei der Wasserstoff enthaltende Frischeinsatzstrom als Strippgasstrom in der Phasentrennung verwendet werden, so dass sich eine besonders vorteilhafte Doppelnutzung ergibt, die auch zu einer weiteren Abreicherung der Flüssigfraktion von leichten Komponenten führt. Insbesondere können dabei die Edukte Wasserstoff und Kohlendioxid für eine Rückführung aus der Flüssigphase ausgetrieben und somit besser genutzt werden. Auch hier kann in einem Rückführstrom ein Inertgaspurge eingesetzt werden. Dieser kann auch beispielsweise einer Druckwechseladsorption oder einem anderen Trennverfahren, bspw. einem Membrantrennverfahren, unterworfen werden.

In einer Ausgestaltung der vorliegenden Erfindung kann der erste Einsatzstrom unter Verwendung eines ersten Teils eines Kohlendioxid enthaltenden Frischeinsatzstroms gebildet werden, und der zweite Einsatzstrom kann unter Verwendung eines zweiten Teils des Kohlendioxid enthaltenden Frischeinsatzstroms gebildet werden. Der Kohlendioxid enthaltende Frischeinsatzstrom kann also aufgeteilt und zu festgelegten oder einstellbaren Anteilen stromauf der ersten Reaktionszone und zwischen der ersten und der zweiten Reaktionszone eingespeist werden. Insbesondere dann, wenn der Kohlendioxid enthaltende Frischeinsatzstrom in Form von flüssigem Kohlendioxid bereitgestellt wird, kann eine besonders gute Kühlung erzielt werden.

In einer Ausgestaltung der vorliegenden Erfindung ist es aber auch möglich, dass zur Bildung des ersten Einsatzstroms nur oder im Wesentlichen nur der Rückführstrom verwendet wird und dass der zweite Einsatzstrom ein Wasserstoff und Kohlendioxid enthaltender Frischeinsatzstrom ist oder aus einem Wasserstoff enthaltenden Frischeinsatzstrom und einem Kohlendioxid enthaltenden Frischeinsatzstrom gebildet wird. Mit der Angabe, dass zur Bildung des ersten Einsatzstroms "im Wesentlichen nur der Rückführstrom" verwendet wird, soll dabei verstanden werden, dass der erste Einsatzstrom zu mehr als 90, 95 oder 99% seines Volumens aus dem Rückführstrom gebildet wird. In einer derartigen Ausgestaltung wird damit im Wesentlichen der gesamte Makeup bzw. Frischeinsatz zwischen der ersten und zweiten Reaktionszone des Reaktorsystems eingespeist.

In Ausgestaltungen der vorliegenden Erfindung kann der Wasserstoff enthaltende Frischeinsatzstrom unter Verwendung einer Elektrolyseeinrichtung bereitgestellt werden, die mit einer Protonen- oder Anionenaustauschmembran ausgebildet ist oder eine Festoxidelektrolysezelle aufweist. Entsprechende Elektrolyseverfahren erlauben eine vereinfachte Bereitstellung des Wasserstoff enthaltenden Frischeinsatzstroms, wie nachfolgend erläutert.

In einer entsprechenden Ausgestaltung kann der Wasserstoff enthaltende Frischeinsatzstrom der Elektrolyseeinrichtung, insbesondere einer Elektrolyseeinrichtung mit einer Protonen- oder Anionenaustauschmembran, auf einem Eintrittsdruckniveau der ersten Reaktionszone oder darüber entnommen werden. Die Elektrolyseeinrichtung wird hierzu typischerweise bei einem Druck betrieben, der beispielsweise ca. 5 bar oberhalb des Eintrittsdruckniveaus der ersten Reaktionszone liegt. Auf diese Weise kann eine Verdichtung und die Bereitstellung eines entsprechenden Verdichters vermieden werden. Ein durch den erhöhten Druck erhöhter Wasserstoffübertritt durch die Membran kann durch die Verwendung einer stabileren bzw. dickeren (gasdichteren) Membran vermieden werden. Auch kann beispielsweise ein Rekombinationskatalysator in der Membranelektrodenanordnung verwendet werden. Alternativ oder zusätzlich kann der Wasserstoff enthaltende Frischeinsatzstrom einer entsprechenden Elektrolyseeinrichtung auch als feuchter Stoffstrom entnommen und in diesem Zustand zur Bildung des Reaktionseinsatzstroms verwendet werden. Hierdurch kann auf einen Trockner verzichtet werden.

Der Kohlendioxid enthaltende Frischeinsatzstrom kann in Ausgestaltungen der Erfindung insbesondere als Flüssigstrom bereitgestellt werden, der flüssig verwendet oder unter Verwendung von Abwärme des Verfahrens verdampft wird. Im Falle der Verwendung eines Flüssigstroms kann dieser insbesondere in flüssigem Zustand auf einen Druck oberhalb des Eintrittsdrucks der ersten Reaktionszone gebracht werden, wodurch auf einen ggf. erforderlichen Gasverdichter verzichtet werden kann. In allen Fällen, auch bei der Verdampfung unter Verwendung von Abwärme des Verfahrens, wird dabei vorteilhafterweise ein Druckniveau eingehalten, das unterhalb des kritischen Drucks von Kohlendioxid (bei ca. 74 bar) liegt.

In Ausgestaltungen der Erfindung kann das Reaktorsystem auf einen Druckniveau von 40 bis 90 bar, insbesondere 50 bis 80 bar, Absolutdruck betrieben werden und/oder die erste Reaktionszone und die zweite Reaktionszone können mit einem Eintrittstemperaturniveau von 190 bis 260 °C, insbesondere 190 bis 230 °C betrieben werden und/oder die erste Reaktionszone kann mit einem Austrittstemperaturniveau von 260 bis 300 °C, insbesondere von 260 bis 280 °C, betrieben werden und/oder die zweite Reaktionszone kann mit einem Austrittstemperaturniveau unterhalb eines Austrittstemperaturniveaus der ersten Reaktionszone betrieben werden. Entsprechende Druck- und Temperaturwerte lassen sich durch die vorliegende Erfindung in besonders vorteilhafter Weise erzielen.

In Ausgestaltungen der Erfindung kann das Reaktorsystem ferner mit einer auf das gesamte Volumen des Reaktorsystems und die gesamten, in das Reaktorsystem eingespeisten Gasmengen bezogenen stündlichen Gasraumgeschwindigkeit von 2.000 bis 12.000 h⁻¹, insbesondere zwischen 2.000 bis 6.000 h⁻¹, betrieben werden.

Grundsätzlich besteht ein Vorteil einer hohen stündlichen Gasraumgeschwindigkeit in der höheren Raumzeitausbeute, allerdings ergibt sich typischerweise eine geringere Katalysator-Lebenszeit. Bei einer niedrigen stündlichen Gasraumgeschwindigkeit sind umgekehrt niedrige Raumzeitausbeuten bei höherer Lebenszeit zu erwarten. Ein Nachteil bei besonders niedrigen stündlichen Gasraumgeschwindigkeiten sind steigende Spurenkomponenten. Die zu wählende stündliche Gasraumgeschwindigkeit ist damit ein Kompromiss zwischen Raumzeitausbeute und Katalysatorlebenszeit (sowie der Bildung von Spurenkomponenten, die den Aufreinigungsaufwand erhöhen). Ja nach Einsatz können unterschiedliche stündliche Gasraumgeschwindigkeiten gewählt werden. Bei konstantem Feed kann eine Optimierung in dem angegebenen Bereich zu erfolgen. Diese liegt insbesondere in einem Bereich von 2.000 bis 6.000 h⁻¹. Bei Verwendung eines flexiblen Feeds können dagegen insbesondere stündliche Gasraumgeschwindigkeiten zwischen 2.000 und 10.000 h⁻¹ eingesetzt werden, wobei unter einem "flexiblen Feed" bzw. Einsatz hier insbesondere ein Einsatz mit schwankendem Wasserstoffanteil, bspw. mit einer Schwankung von mehr als 10 Prozentpunkte um einen Mittelwert, verstanden wird. Eine derartige Schwankung kann sich insbesondere ergeben, wenn Wasserstoff aus einer Elektrolyse mit fluktulierender Stromverfügbarkeit und ggf. ohne Wasserstoffpuffer stammt. In einem derartigen Fall kann insbesondere ein flexibles Rezirkulationsgebläse und ein Flüssigspeicher vor einer Rohmethanolaufreinigung in einer oder mehreren Kolonnen zum Einsatz kommen.

In Ausgestaltungen der Erfindung kann das Reaktorsystem derart betrieben wird, dass ein Verhältnis von Wasserstoff zu Kohlendioxid am Eintritt der ersten Reaktionszone zwischen 3 und 10, insbesondere zwischen 7 bis 9, liegt, und/oder dass ein Verhältnis von Wasserstoff zu Kohlendioxid am Eintritt der zweiten Reaktionszone zwischen 3 bis 10, insbesondere zwischen 7 bis 9, liegt, und dass der eingesetzte Wasserstoff zum eingesetzten Kohlendioxid in einem Verhältnis von 2,8 bis 3,3, insbesondere von 2,9 bis 3,1, liegt. Mit den Verhältnissen in den entsprechenden Einsatzströmen wird die Zusammensetzung des Recyclestroms vorgegeben.

Bei mehr Wasserstoff im Einsatz wird die Alterung des Katalysators reduziert, da Kohlendioxid- und Wasser-Partialdrücke über das gesamte Katalysatorbett reduziert, werden. Beide Komponenten sind maßgeblich für die Alterung verantwortlich. Außerdem sind niedrigere Partialdrücke von Kohlendioxid zu erwarten und dadurch eine niedrigere Nebenproduktbildung. Es ergeben sich hierbei insbesondere auch geringere Kohlenstoffverluste durch Purgeströme. Ein Nachteil von mehr Wasserstoff im Einsatz besteht im höheren Energieaufwand für Recyclekompressoren.

Bei niedrigen Verhältnissen von Wasserstoff zu Kohlendioxid (im Bereich von 3 bis 5) kann eine maximale Raumzeitausbeutung und niedrigere Kompressorleistung erzielt werden. Im Rahmen der vorliegenden Erfindung wurden Werte von 5 bis 10, insbesondere bevorzugt von 6 bis 10, als besonders vorteilhaft erkannt, da in diesem Fall ein einfacheres Kolonnensetup mit niedrigeren Spurenkomponenten möglich ist. Das Verhältnis wird insbesondere beeinflusst durch Kohlendioxidverluste (z.B. beim Strippen) und durch die Rückführraten des Wasserstoffs (z.B. dein Einsatz einer Druckwechseladsorption oder anderen Trennung zur Rückgewinnung von Wasserstoff in einem Purgestrom).

In einer Ausgestaltung der vorliegenden Erfindung wird die Flüssigphase aus der Phasentrennung zumindest zu einem Teil in eine Trenneinheit, insbesondere eine Rektifikationskolonne, eingespeist, wobei unter Verwendung einer Sumpfflüssigkeit der Trenneinheit, die überwiegend Wasser enthalten kann, ein flüssiger Wasserstrom und unter Verwendung eines oder mehrerer, in einem oberen Bereich der Trenneinheit abgezogener Gase oder Gasgemische ein flüssiger Methanolstrom und ein gasförmiger Restgasstrom, der überwiegend oder ausschließlich leichter als Methanol siedende Komponenten aufweist, gebildet werden. Der Methanolstrom kann insbesondere unter Verwendung eines eine oder mehrere Trennstufen oberhalb des Kopfes der Kolonne abgezogener Gase oder Gasgemische gebildet werden. Ferner wird in dieser Ausgestaltung der Trenneinheit ein flüssiger Seitenstrom entnommen, der überwiegend oder ausschließlich zwischen Wasser und Methanol siedende Komponenten enthält, insbesondere Ethanol. Zum Begriff "überwiegend" sei auf die obigen Erläuterungen verwiesen. Der Begriff "Wasserstrom" bzw. "Methanolstrom" soll daher auch an entsprechenden Komponenten reiche Gemische bezeichnen.

Eine Anlage zur katalytischen Herstellung von Methanol, die ein Reaktorsystem mit einer ersten Reaktionszone und einer zweiten Reaktionszone aufweist, ist ebenfalls Gegenstand der vorliegenden Erfindung, wobei die erste Reaktionszone und die zweite Reaktionszone jeweils für einen adiabaten Betrieb eingerichtet sind und jeweils eine katalytische Reaktionszone aufweisen, und wobei die Anlage Mittel aufweist, die dafür eingerichtet sind, zumindest einen Teil eines aus der ersten Reaktionszone abströmenden Gasgemischs in die zweite Reaktionszone zu überführen, einen ersten Einsatzstrom auf einem ersten Temperaturniveau stromauf der ersten Reaktionszone in das Reaktorsystem einzuspeisen und einen zweiten Einsatzstrom auf einem zweiten Temperaturniveau zwischen der ersten Reaktionszone und der zweiten Reaktionszone in das Reaktorsystem eingespeist wird, wobei der erste Temperaturbereich oberhalb des zweiten Temperaturbereichs liegt.

Zu weiteren Merkmalen und den Vorteilen einer entsprechenden Anlage sei auf die obigen Erläuterungen zu dem erfindungsgemäßen Verfahren und seiner Ausgestaltungen ausdrücklich verwiesen, zu dessen Durchführung eine entsprechende Anlage eingerichtet sein kann.

Nochmals zusammengefasst bestehen die Vorteile der vorliegenden Erfindung insbesondere in geringen Erstellungskosten. Beispielsweise ist bei Wahl geeigneter Elektrolysebedingungen kein Wasserstoffverdichter erforderlich und/oder ein Trockner kann entfallen. In Ausgestaltungen der Erfindung ist nur eine Trenneinheit erforderlich. Das Reaktorsystem bedarf keiner Kühlung und keiner entsprechenden Ausgestaltung, beispielsweise in Form eines Rohrbündelreaktors. Es ist keine Installation einer reversen Wassergasshift erforderlich und auf die Verwendung komplexer Wärmeübertrager, Doppelhüllen und zwischengeschalteter Abscheider kann verzichtet werden.

Die im Rahmen der Erfindung vorgesehene Zwischeneinspeisung führt zu besseren Reaktionsgleichgewichten verglichen mit einem Reaktorsystem ohne solche. Aufgrund der geringeren Temperatur in der zweiten Reaktionszone ist die Konversion aber jener in einem gekühlten Reaktorsystem vergleichbar. Durch die Temperaturreduktion kommt es zudem zu einer verringerten Kohlenmonoxidbildung. Da Kohlenmonoxid maßgeblich zur Nebenproduktbildung beiträgt, wird diese durch die Zwischeneinspeisung ebenfalls reduziert.

Weitere Ausgestaltungen der Erfindung können die thermische oder stoffliche Nutzung des Purgegases oder eines Restgases aus der Trenneinheit, beispielsweise in einem befeuerten Heizer, einer katalytischen Einheit oder einer Hochtemperaturbrennstoffzelle umfassen. Ein (chemisches) Wärmepumpensystem kann verwendet werden, um in einer Niedertemperaturelektrolyse auf niedrigem Temperaturniveau anfallende Wärme oder aus dem Reaktorsystem (nach ggf. anderen Kühlschritten) noch vorhandene Wärme, bspw. für einen Sumpfverdampfer der Trenneinheit, zu nutzen.

Wasserstoff kann grundsätzlich auch aus externen Quellen stammen. Kohlendioxid kann beispielsweise in einer Carbon Capture gewonnen werden. Das erhaltene Methanol kann weiter aufgereinigt und/oder für Treibstoffzwecke oder zur Umsetzung zu Treibstoffkomponenten wie Methyl-tert-Butylether genutzt werden.

Die Erfindung wird nachfolgend unter Bezugnahme auf die beigefügten Zeichnungen weiter erläutert, die Ausgestaltungen der vorliegenden Erfindung veranschaulichen.

### Kurze Beschreibung der Zeichnungen

Figur 1 veranschaulicht ein Verfahren gemäß einer Ausgestaltung der Erfindung.
Figur 2 veranschaulicht ein Verfahren gemäß einer Ausgestaltung der Erfindung.
Figur 3 veranschaulicht ein Verfahren gemäß einer Ausgestaltung der Erfindung.

In den Figuren sind einander baulich und/oder funktional entsprechende Komponenten mit identischen Bezugszeichen angegeben und werden lediglich der Übersichtlichkeit halber nicht wiederholt erläutert.

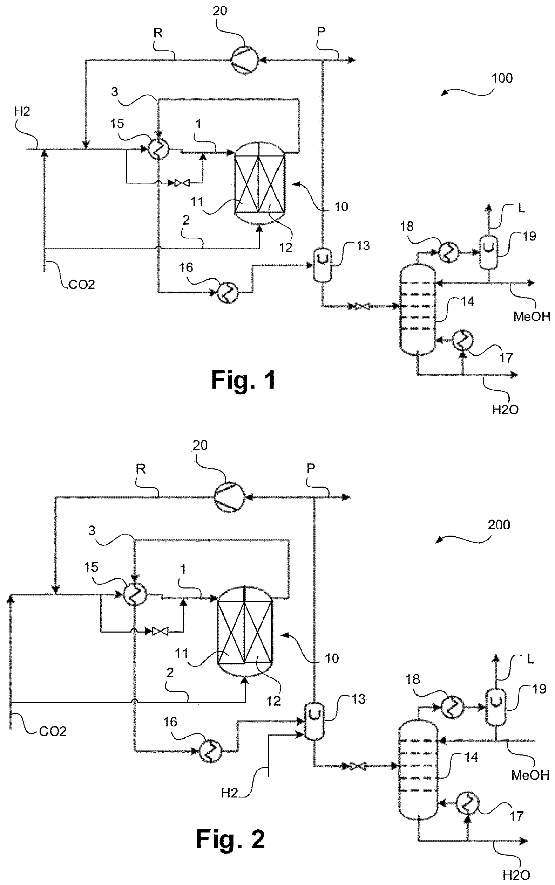

In den Figuren 1 bis 3 sind jeweils Verfahren gemäß Ausgestaltungen der Erfindung anhand von vereinfachten Prozessflussdiagrammen veranschaulicht und jeweils insgesamt mit 100, 200 bzw. 300 bezeichnet. Die nachfolgenden Erläuterungen betreffen entsprechende Vorrichtungen in gleicher Weise, wobei Verfahrensschritte und Vorrichtungskomponenten bzw. Apparate auch teilweise mit identischen Bezugszeichen angegeben sind.

In den Figuren 1 bis 3 ist jeweils ein Reaktorsystem 10 mit einer ersten Reaktionszone 11 und einer zweiten Reaktionszone 12 dargestellt, wobei die erste Reaktionszone 11 und die zweite Reaktionszone 12 adiabat betrieben werden. Zumindest ein Teil eines aus der ersten Reaktionszone 11 abströmenden Gasgemischs wird dabei in die zweite Reaktionszone 12 überführt. Es wird jeweils ein erster Einsatzstrom 1 auf einem ersten Temperaturniveau stromauf der ersten Reaktionszone 11 in das Reaktorsystem 10 eingespeist und ein zweiter Einsatzstrom wird auf einem zweiten Temperaturniveau zwischen der ersten Reaktionszone 11 und der zweiten Reaktionszone 12 in das Reaktorsystem 10 eingespeist. Der erste Temperaturbereich liegt oberhalb des zweiten Temperaturbereichs.

In sämtlichen Ausgestaltungen 100, 200 und 300 wird ein aus der zweiten Reaktionszone 12 abströmendes Gasgemisch 3 zunächst durch einen Wärmeübertrager 15 geführt und in diesem gegen zumindest einen Teil des ersten Einsatzstroms 1 abgekühlt. Zur Einstellung einer Temperatur des ersten Einsatzstroms kann dabei ein nicht gesondert bezeichneter Bypass verwendet werden. Das Gasgemisch 3 wird in einem weiteren Wärmeübertrager 16 zur weiteren Kühlung eingespeist und unter Erhalt einer Gasfraktion und einer Flüssigfraktion einer Phasentrennung 13 in einem entsprechenden Behälter unterworfen. Unter Verwendung eines Teils der Gasfraktion wird ein Rückführstrom R gebildet, der mittels eines Verdichters bzw. Gebläses auf einen Einspeisedruck in das Reaktorsystem 10 bzw. der ersten Reaktionszone 11 gebracht wird. Der erste Einsatzstrom wird also in Ausgestaltungen 100, 200 und 300 (zumindest) unter Verwendung des Rückführstroms gebildet. Von der Gasphase wird ferner im dargestellten Beispiel ein Purgestrom P ausgeschleust.

In den Ausgestaltungen 100 und 200 gemäß den Figuren 1 und 2 wird der erste Einsatzstrom 1 unter Verwendung eines Wasserstoff H2 enthaltenden Frischeinsatzstroms gebildet, wobei in der Ausgestaltung 100 gemäß Figur 1 der erste Einsatzstrom 1 unter Verwendung des Wasserstoff enthaltenden Frischeinsatzstroms H2 und des Rückführstroms R gebildet wird, in der Ausgestaltung 200 gemäß Figur 2 dagegen der Wasserstoff enthaltende Frischeinsatzstrom H2 als Strippgas in die Phasentrennung 13 eingespeist wird und daher zumindest ein Teil des Wasserstoffs in dem Wasserstoff enthaltenden Frischeinsatzstrom in der Phasentrennung 13 in die Gasfraktion und hieraus in den Rückführstrom überführt wird. In beiden Fällen wird dabei der erste Einsatzstrom unter Verwendung eines ersten Teils eines Kohlendioxid enthaltenden Frischeinsatzstroms CO2 gebildet, und der zweite Einsatzstrom wird unter Verwendung eines zweiten Teils des Kohlendioxid enthaltenden Frischeinsatzstroms CO2 gebildet bzw. stellt diesen Teil dar.

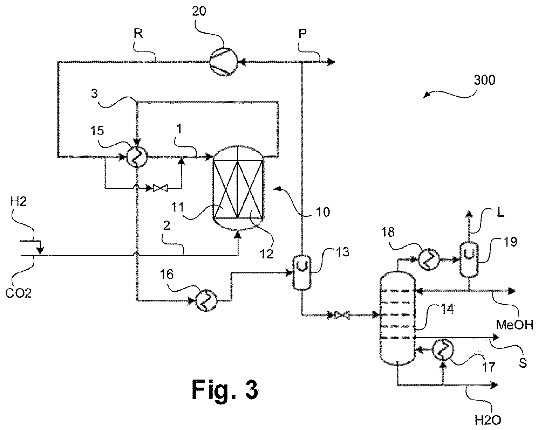

Abweichend dazu wird in der Ausgestaltung 300 gemäß Figur 3 zur Bildung des ersten Einsatzstroms 1 nur der Rückführstrom R verwendet, der zweite Einsatzstrom 2 ist dagegen ein Wasserstoff und Kohlendioxid enthaltender Frischeinsatzstrom bzw. ist aus einem Wasserstoff enthaltenden Frischeinsatzstrom H2 und einem Kohlendioxid enthaltenden Frischeinsatzstrom CO2 gebildet.

In den Figuren ist veranschaulicht, dass die Flüssigphase aus der Phasentrennung 13 in eine Trenneinheit 14, insbesondere eine Rektifikationskolonne, eingespeist wird, der die Wärmeübertrager (Heizer bzw. Kühler) 17 und 18 sowie ein Abscheider 19 zugeordnet sind. Unter Verwendung einer Sumpfflüssigkeit aus der Trenneinheit 14 wird dabei ein Wasserstrom H2O, unter Verwendung eines Gases aus einem oberen Bereich der Trenneinheit 14 dagegen ein flüssiger Methanolstrom MeOH und ein Restgasstrom L aus leichter als Methanol siedenden Komponenten erhalten. Wie in Figur 3 veranschaulicht, aber grundsätzlich in allen veranschaulichten Ausgestaltungen möglich, wird der Trenneinheit 14 ein Seitenstrom S entnommen, der zwischen Wasser und Methanol siedende Komponenten, insbesondere Ethanol, enthalten kann.

## Patentansprüche

1. Verfahren (100, 200, 300) zur katalytischen Herstellung von Methanol, wobei ein Reaktorsystem (10) mit einer ersten Reaktionszone (11) und einer zweiten Reaktionszone (12) verwendet wird, wobei die erste Reaktionszone (11) und die zweite Reaktionszone (12) adiabat betrieben werden, wobei zumindest ein Teil eines aus der ersten Reaktionszone (11) abströmenden Gasgemischs in die zweite Reaktionszone (12) überführt wird, wobei ein erster Einsatzstrom auf einem ersten Temperaturniveau stromauf der ersten Reaktionszone (11) eingespeist wird, wobei ein zweiter Einsatzstrom auf einem zweiten Temperaturniveau zwischen der ersten Reaktionszone (11) und der zweiten Reaktionszone (12) in das Reaktorsystem (10) eingespeist wird, und wobei der erste Temperaturbereich oberhalb des zweiten Temperaturbereichs liegt.

2. Verfahren (100, 200, 300) nach Anspruch 1, wobei zumindest ein Teil eines aus der zweiten Reaktionszone (12) abströmenden Gasgemischs unter Erhalt einer Gasfraktion und einer Flüssigfraktion einer Phasentrennung (13) unterworfen wird, wobei unter Verwendung zumindest eines Teils der Gasfraktion ein Rückführstrom gebildet wird, und wobei der erste Einsatzstrom unter Verwendung des Rückführstroms gebildet wird.

3. Verfahren (100, 200, 300) nach Anspruch 2, wobei zumindest ein Teil des aus der zweiten Reaktionszone (12) abströmenden Gasgemischs gegen zumindest einen Teil des ersten Einsatzstroms abgekühlt wird.

4. Verfahren (100, 200) nach einem der Ansprüche 2 oder 3, wobei der erste Einsatzstrom unter Verwendung eines Wasserstoff enthaltenden Frischeinsatzstroms gebildet wird.

5. Verfahren (100) nach Anspruch 4, bei dem der erste Einsatzstrom unter Verwendung des Wasserstoff enthaltenden Frischeinsatzstroms und des Rückführstroms gebildet wird.

6. Verfahren (200) nach Anspruch 4, wobei der Wasserstoff enthaltende Frischeinsatzstrom in die Phasentrennung (13) eingespeist wird und wobei zumindest ein Teil des Wasserstoffs in dem Wasserstoff enthaltenden Frischeinsatzstrom in der Phasentrennung (13) in die Gasfraktion und hieraus in den Rückführstrom überführt wird.

7. Verfahren (100, 200) nach einem der Ansprüche 2 bis 6, wobei der erste Einsatzstrom unter Verwendung eines ersten Teils eines Kohlendioxid enthaltenden Frischeinsatzstroms gebildet wird, und wobei der zweite Einsatzstrom unter Verwendung eines zweiten Teils des Kohlendioxid enthaltenden Frischeinsatzstroms gebildet wird.

8. Verfahren (300) nach einem der Ansprüche 2 oder 3, wobei zur Bildung des ersten Einsatzstroms nur der Rückführstrom verwendet wird und wobei der zweite Einsatzstrom ein Wasserstoff und Kohlendioxid enthaltender Frischeinsatzstrom ist oder aus einem Wasserstoff enthaltenden Frischeinsatzstrom und einem Kohlendioxid enthaltenden Frischeinsatzstrom gebildet wird.

9. Verfahren (100, 200, 300) nach einem der Ansprüche 4 bis 8, bei dem der Wasserstoff enthaltende Frischeinsatzstrom unter Verwendung einer Elektrolyseeinrichtung bereitgestellt wird, die als Protonen- oder Anionenaustauschelektrolyseeinrichtung ausgebildet ist oder eine Festoxidelektrolysezelle aufweist.

10. Verfahren (100, 200, 300) nach Anspruch 9, wobei der Wasserstoff enthaltende Frischeinsatzstrom der Elektrolyseeinrichtung auf einem Eintrittsdruck der ersten Reaktionszone (11) und/oder als feuchter Stoffstrom entnommen wird.

11. Verfahren (100, 200, 300) nach einem der Ansprüche 7 oder 8, bei dem der Kohlendioxid enthaltende Frischeinsatzstrom als Flüssigstrom bereitgestellt wird, der flüssig verwendet oder unter Verwendung von Abwärme des Verfahrens (100, 200, 300) verdampft wird.

12. Verfahren (100, 200, 300) nach einem der vorstehenden Ansprüche, bei dem das Reaktorsystem (10) auf einen Druckniveau von 40 bis 90 bar oder 50 bis 80 bar Absolutdruck betrieben wird und/oder bei dem die erste Reaktionszone (11) und die zweite Reaktionszone (12) mit einem Eintrittstemperaturniveau von 190 bis 260 °C oder 190 bis 230 °C betrieben werden und/oder bei dem die erste Reaktionszone (11) mit einem Austrittstemperaturniveau von 260 bis 300 °C oder 260 bis 280 °C betrieben wird und/oder bei dem die zweite Reaktionszone (12) mit einem Austrittstemperaturniveau unterhalb eines Austrittstemperaturniveaus der ersten Reaktionszone (11) betrieben wird.

13. Verfahren (100, 200, 300) nach einem der vorstehenden Ansprüche, bei dem das Reaktorsystem (10) mit einer auf das gesamte Volumen des Reaktorsystems (10) und die gesamten, in das Reaktorsystem (10) eingespeisten Gasmengen bezogenen stündlichen Gasraumgeschwindigkeit von 2.000 bis 12.000 h⁻¹ oder 2.000 bis 10.000 h⁻¹ betrieben wird.

14. Verfahren (100, 200, 300) nach einem der vorstehenden Ansprüche, bei dem das Reaktorsystem (10) derart betrieben wird, dass ein Verhältnis von Wasserstoff zu Kohlendioxid am Eintritt der ersten Reaktionszone (11) bei 3 bis 10 oder 4 bis 9 und/oder am Eintritt der zweiten Reaktionszone (12) bei 3 bis 10 oder 4 bis 9 und/oder in dem zweiten Einsatzstrom bei 2,8 bis 3,3 oder 2,9 bis 3,1 liegt.

15. Anlage zur katalytischen Herstellung von Methanol, die ein Reaktorsystem (10) mit einer ersten Reaktionszone (11) und einer zweiten Reaktionszone (12) aufweist, wobei die erste Reaktionszone (11) und die zweite Reaktionszone (12) jeweils für einen adiabaten Betrieb eingerichtet sind, und wobei die Anlage Mittel aufweist, die dafür eingerichtet sind, zumindest einen Teil eines aus der ersten Reaktionszone (11) abströmenden Gasgemischs in die zweite Reaktionszone (12) zu überführen, einen ersten Einsatzstrom auf einem ersten Temperaturniveau stromauf der ersten Reaktionszone (11) in das Reaktorsystem (10) einzuspeisen und einen zweiten Einsatzstrom auf einem zweiten Temperaturniveau zwischen der ersten Reaktionszone (11) und der zweiten Reaktionszone (12) in das Reaktorsystem (10) einzuspeisen, wobei der erste Temperaturbereich oberhalb des zweiten Temperaturbereichs liegt.
